# EUROPEAN PATENT SPECIFICATION

(11) **EP 1 073 594 B1**
(45) Date of publication and mention of the grant of the patent: **23.07.2003**
(21) Application number: 99918898.0
(22) Date of filing: 29.04.1999
(51) Int. Cl.: B65D 83/14

(54) **METERED DOSE VALVE FOR PHARMACEUTICAL AEROSOL**
DOSIERVENTIL FÜR PHARMAZEUTISCHES AEROSOL
VALVE DE DOSAGE POUR AEROSOL PHARMACEUTIQUE

(30) Priority: 30.04.1998 US 83829 P
(43) Date of publication of application: 07.02.2001
(73) Proprietor: 3M Innovative Properties Company, Saint Paul, MN 55133-3427 (US)
(72) Inventor: ALBAND, Todd, D., Saint Paul, MN 55133-3427 (US); DUPONT, Richard, L., Saint Paul, MN 55133-3427 (US); ROSS, Danna, L., Saint Paul, MN 55133-3427 (US); SZKUDLAREK, Beth, A., Saint Paul, MN 55133-3427 (US); ZINN-WARNER, Alexis, Saint Paul, MN 55133-3427 (US)
(74) Representative: VOSSIUS & PARTNER
(86) International application number: US9909314
(87) International publication number: WO99055600

(56) References cited:
- EP-A- 0 373 753
- EP-A- 0 673 857
- EP-A- 0 803 449
- WO-A-96/32345
- GB-A- 432 818
- GB-A- 798 683
- GB-A- 2 087 840
- GB-A- 2 323 351
- US-A- 3 669 407

## Description

This invention relates to pharmaceutical aerosol valves and in particular to metered dose dispensing valves for administration of metered quantities of medicinal aerosol formulation.

The use of aerosols to administer medicament has been known for several decades. Such aerosol formulations generally comprise medicament, one or more propellants and surfactant and/or a solvent, such as ethanol. In the past the most commonly used aerosol propellants have been propellant 11 (CCl₃F) and/or propellant 114 (CF₂ClCF₂Cl) with propellant 12 (CCl₂F₂). However, these propellants are chlorofluorocarbons which are believed to provoke degradation of stratospheric ozone and recently so-called "ozone-friendly" propellants have been proposed, particularly hydrogen-containing fluorocarbons, such as propellant 134a (CF₃CH₂F) and propellant 227 (OF₃CHFCF₃).

One of the main types of medicinal aerosol is for the administration of medicament by inhalation. This route of administration is particularly suitable for medicaments, such as, anti-allergics, bronchodilators and antiinflammatory steroids for use in the treatment of respiratory disorders, such as, asthma. However, inhalation is also used as an administration route for other medicaments e.g. analgesics, anti-infectives, hormones, therapeutic proteins and peptides etc.

Pharmaceutical aerosols for inhalation generally comprise a container or vial of the aerosol formulation equipped with a metered dose dispensing valve. The valve typically comprises a valve ferrule for supporting the valve components and attachment to the container, a metering chamber, a valve stem and associated elastomeric seals and one or more springs. The valve stem passes through the ferrule and metering chamber in sealing engagement. The valve is generally configured such that when the valve stem is in its non-dispensing position aerosol formulation in the container may enter the metering chamber; as the valve stem is moved towards its dispensing position (normally by depressing the valve stem inwardly) communication between the metering chamber and container is prevented; and in its dispensing position contents of the metering chamber may pass through a side port in the valve stem and exit via a bore in the valve stem.

In the context of inhalation drug delivery, the aerosol container is used in a press-and-breathe or breath-actuated actuator. The valve stem is located in a nozzle block which defines an expansion chamber and an orifice which directs the formulation towards the mouthpiece of the actuator. The aerosol is fired by the patient depressing the aerosol container in the case of a press-and-breathe actuator or by mechanical actuation in response to inhalation in the case of a breath-actuated actuator.

It will be appreciated that the aerosol formulation, aerosol valve and actuator each play an important part in obtaining optimum pharmaceutical properties from the product.

The aerosol formulation and valve design should ensure that a homogenous formulation is delivered to the metering chamber throughout the life of the product. The valve design should reliably deliver the precise metered volume of homogenous formulation for every actuation throughout the life of the product, which is often in excess of 200 doses. There should be no blockage of the valve or actuator which could impair or prevent medicament delivery.

There are many different designs of metered dose aerosol valves. The majority employ a metering chamber and valve stem but have different constructional features e.g. to compensate for the properties of particular aerosol formulations, such as whether a suspension of drug particles sink or float; to provide different routes for entry of formulation into the metering chamber; to provide means to allow pressure filling the aerosol container during manufacture; to provide means for completely emptying the container, etc.

The materials used in the constructions of the individual components of the valve are typically as follows:
a) metal pressings and deep drawings; e.g. ferrules, stems etc.
b) thermoplastic injection mouldings e.g. stems, bodies etc.,
c) elastomeric seals
d) wound wire springs

The raw material for metal pressing and deep drawing components usually comes in the form of a strip e.g. aluminium or stainless steel, typically in the range of 0.25mm to 0.5mm thick, heat treated to increase the malleability to allow working. The raw strip is fed into sequential press tooling where the material is clamped securely over a cylindrical die. A mating punch then impacts onto the strip material and carries it down into the die. The act of clamping the material minimises the rippling effect, which would otherwise occur. The clearance between the punch and die is such that, on the side walls, it is less than the initial thickness of the strip. The net result is that the material is ironed or thinned.

To achieve the final desired shape, many such operations may be carried out. With most metals, if they are 'worked' in a cold condition, they become harder and more brittle. Each stage of the drawing operation has to be designed such that the degree of deformation is achieved while remaining within the material's capability of withstanding it.

In highly worked materials, such as found in stems, micro-fractures occur on the surface, initiating at grain boundaries. If the component is used in a dynamic situation, the cracking effect on the surface can increase the frictional characteristics. In many cases deep drawings are processed to both improve surface finish and remove sharp edges.

The raw materials for thermoplastic injection mouldings come in the form of granules of polymer. In moulding, the shape of the final component is achieved through the application of heat and pressure in a controlled manner. In general this is achieved using a system consisting of two elements, the injection moulding machine and the mould tool. The role of the machine is to preplasticise the material, by applying both heat and shear energy to a bulk of the polymer. At a pre-determined point, the material is forced from the machine into the mould tool under extreme pressure (600 to 800 bar).

The mould tools are precision assemblies of hardened steel components, which open and close every 10 to 20 seconds. Coupled with the fact that the molten plastic has a very low viscosity, at various points, plastic can escape or seep where the mould segments meet. This results in 'flash'; depending on where this occurs, valve function could be impaired.

Other typical defects result from the flow (or lack thereof) of the molten plastic. Components can be encountered which are not completely filled and defined. This can either be as a result of incomplete filling or entrapped gas/air.

The selection of metal or plastic as the material for a particular valve component will depend upon a variety of factors e.g. complexity of the shape of the components, strength characteristics required and the behaviour of the material in contact with the aerosol formulation and the associated components of the valve.

Valve stems have conventionally been constructed by injection moulding thermoplastics and by deep drawing metal. The potential problems associated with thermoplastics include selection of a material which has a suitable viscosity for moulding with low flash, no shrinkage after moulding, no change in dimension with temperature, rigid without being brittle, will not swell when contacted with the aerosol formulation, no extractables when in contact with the aerosol formulation, no drug updake when in contact with the aerosol formulation, low moisture uptake/transfer, must seal well with the elastomeric seals with which it is in contact. Stainless steel has been viewed as the most inert option in dealing with compatibility problems between the valve and the formulation and possesses the required mechanical strength and resistance to moisture. However, there is a limit to the complexity and geometric accuracy of shapes produced by metal pressing and drawing.

The use of formulations containing propellant 134a and/or propellant 227 has placed additional constraints upon the design of metered dose aerosol valves. It has been necessary to develop new elastomeric seals and the formulations tend to have a higher vapour pressure. Furthermore, some formulations, particularly solution formulations, contain significant amounts of ethanol, which is relatively non-volatile compared to the propellants and may give rise to residue in the valve stem and/or actuator following release of a metered dose formulation.

It has been found that formulations, particularly solution formulations, comprising medicament in ethanol and propellant 134a and/or propellant 227 may give rise to blockage in valve stems constructed of deep drawn metal e.g. stainless steel and the associated actuators in which they are used. Actuators for solution aerosol formulation tend to have a small orifice size e.g. about 0.3mm to ensure generation of droplets of optimum size for inhalation. Investigation has revealed there are several factors associated with conventional deep drawn valve stems that contribute to the blockage problem.

EP 0 803 449 A1 discloses a dispensing apparatus for dispensing a product from a pressurized container comprising a metering valve. The valve comprises a valve body defining a metering chamber, an outer annular seal closing an outer end of the chamber, an inner annular seal at an inner end of the chamber and a valve stem extending in sliding sealed relation through the chamber and engaging at its inner end a valve stem member. Spring means are provided urging the valve stem member into sealing engagement with the inner seal such that the chamber is sealed, the valve stem being manually movable against the action of the spring means to unseat the valve stem member into a first position in which a fluid inlet path is opened into the chamber to enable filling of the chamber. The valve stem being movable further to a second position in which the fluid inlet path is closed and a fluid outlet path from the chamber is opened to enable the product to be dispensed from the chamber.

An object of the present invention is to provide a metered dose aerosol valve suitable for use with formulations comprising a hydrogen-containing fluorocarbon propellant having a metal valve stem in which blockage problems are substantially reduced. This object is achieved with the features of the claims.

According to the present invention there is provided a blockage resistant metered dose aerosol product comprising an aerosol container having an aerosol formulation therein comprising medicament in one or more hydrogen-containing propellants, said container being equipped with a aerosol valve comprising a metering chamber and a valve stem extending into the metering chamber in sealing engagement therewith, the valve stem having an outer end projecting from the metering chamber, said outer end having a central outlet channel and a side port in communication with the outlet channel, the valve stem being movable between a non-dispensing position in which said side port is located outside the metering chamber and a dispensing position in which said side port is located within the metering chamber to facilitate dispensing of contents from the metering chamber through said side port and outlet channel, and wherein at least said outer end of the valve stem is made of metal, said side port joins the outlet channel substantially at the inner end thereof and the outlet channel has a constant cross-section throughout its length from the region of the side port to its outlet. In addition, the present invention provides blockage resistant medicinal aerosol products wherein the medicament is dissolved in the formulation, wherein the formulation comprises ethanol and a hydrofluorocarbon propellant selected from P134a (CF₃CH₂F) P227 (CF₃CHFCF₃) and mixtures thereof.

It is believed that blockage resistance is substantially improved in these stems for a number of reasons. For example, one important reason is that the valve stem design of the present invention has a much lower internal volume as compared to conventional deep-drawn stems. Another reason is that the constant cross-section avoids the internal obstructions caused by the end curl and side port burr of conventional deep drawn valve stems.

The invention also provides metal valve stems, preferably stainless steel, with improved corrosion resistance and surface properties (e.g., low friction) by bright annealing the stems, as well as by surface treatment with nitric acid, citric acid, and barrelling/tumbling the stems. Bright annealing is also believed to improve blockage resistance by improving the interior surface finish of the valve stem.

In accordance with a further aspect of the invention there is provided a metered dose inhaler comprising an actuator and an aerosol product as defined above. The actuator comprising a nozzle block and a mouthpiece, the nozzle block defining an aperture for accommodating the end of the valve stem and an orifice in communication with the aperture directed towards the mouthpiece, the orifice having a diameter of less than 0.4mm, preferably about 0.3mm.

The invention will now be described with reference to the accompanying drawings in which:
Figure 1 represents a cross-section through a metered dose aerosol valve,
Figure 2 represents a cross-section through a deep drawn metal valve stem of the prior art,
Figure 3 represents a cross-section through a valve stem of the invention and,
Figure 4 represents a cross-section through a press-and-breathe actuator.

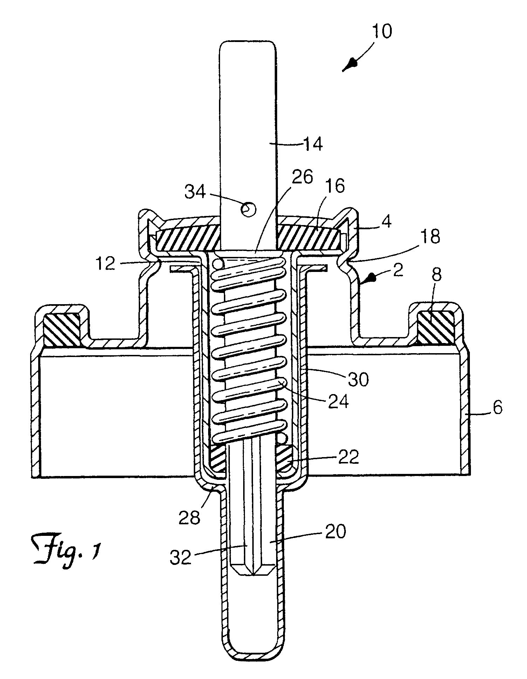

Figure 1 illustrates a metered dose aerosol valve comprising a ferrule, generally shown at (2) having a central portion (4) retaining the components of the valve. The ferrule has a circumferential flange (6) for crimping around the neck of an aerosol container (not shown) to secure the valve to the container. A gasket (8) is positioned to be clamped between the neck of the aerosol container and ferrule ensuring a gas-tight seal.

The valve comprises a valve stem generally shown at (10) extending through a central aperture in the ferrule through a metering chamber (12). The outer end (14) of the valve stem projects from the metering chamber (12) and is in sealing engagement with elastomeric diaphragm (16). The metering chamber (12) and diaphragm (16) are secured in place by a crimp (18) in the ferrule (2).

The inner end (20) of the valve stem projects through the metering chamber (12) in sealing engagement with a tank seal (22). A spring (24) is positioned within the metering chamber, one end abutting the tank seal (22) and the other end abutting a flange (26) extending around an intermediate portion of the valve stem. The spring (24) urges the valve stem to its non-dispensing position as shown in Figure 1. A bottle emptier (28) extends around the metering chamber (12) terminating towards the diaphragm (16). The bottle emptier and wall of the metering chamber defines a capillary passage (30) through which aerosol formulation reaches the metering chamber.

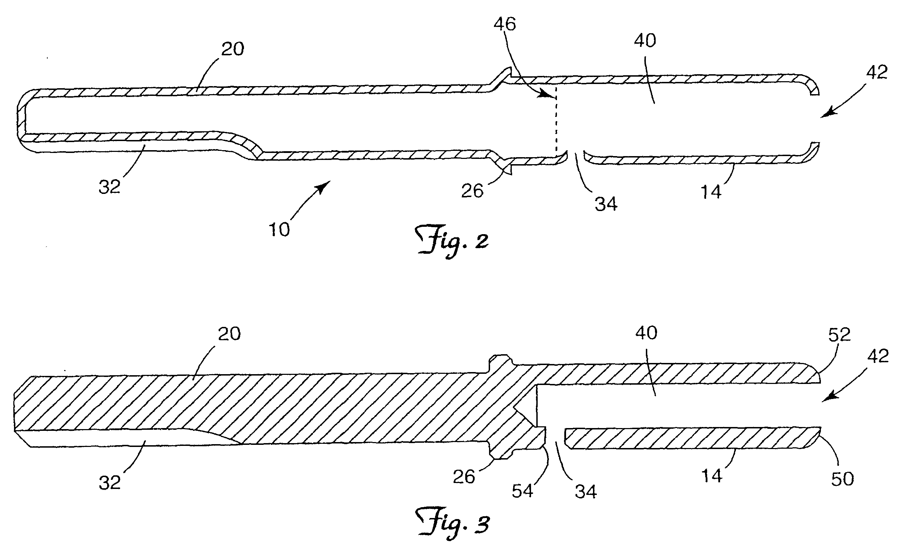

In its non-dispensing position, when the valve is inverted relative to the position shown in Figure 1, contents of the aerosol container enter the metering chamber (12) by passing through the capillary passage (30) and groove (32) on the inner end (20) of the valve stem. When the valve stem is moved to its dispensing position (not shown) the groove (32) passes outside the tank seal (22) and thus the inner end of the valve stem completes a seal preventing flow of aerosol formulation in to and out of the metering chamber through the filling end. In its dispensing position, the side port (34) of the valve stem passes through the diaphragm into the metering chamber. The side port (34) is in communication with a central outlet passage (Figures 2 and 3) allowing the contents of the metering chamber to be dispensed from the valve.

Figure 2 of the accompanying drawings represents a cross-section through a deep drawn valve stem which has been used in a metered dose dispensing valve of the type shown in Figure 1. The valve stem (10) comprises an outer portion (14), an inner portion (20), a flange (26), groove (32) and side port (34) as described with reference to Figure 1.

The valve stem of Figure 2 is hollow having a central passage (40). The outlet end (42) of the valve stem is curved inwardly restricting the size of the central chamber (40). The function of the inwardly curved wall is to facilitate assembly of the diaphragm on the valve stem, to provide additional strength, to facilitate insertion into the nozzle block, and to provide a larger surface area to contact the nozzle block when inserted into the aperture of a nozzle block of an actuator.

The side port (34) is generally formed by punching. This operation results in the outer surface of the valve stem being dished around the side port (34). This dishing is desirable since it ensures there is no sharp edge that contacts the diaphragm during movement of the valve stem Sharp edges can cause erosion of the diaphragm resulting in leakage and/or pieces of diaphragm contaminating the aerosol formulation.

The punching operation also results in a circumferential projection or burr being formed around the side port within the valve stem. In some cases, the metal punched out to form the side port may not be completely removed leaving a large burr projecting inwardly into the valve stem.

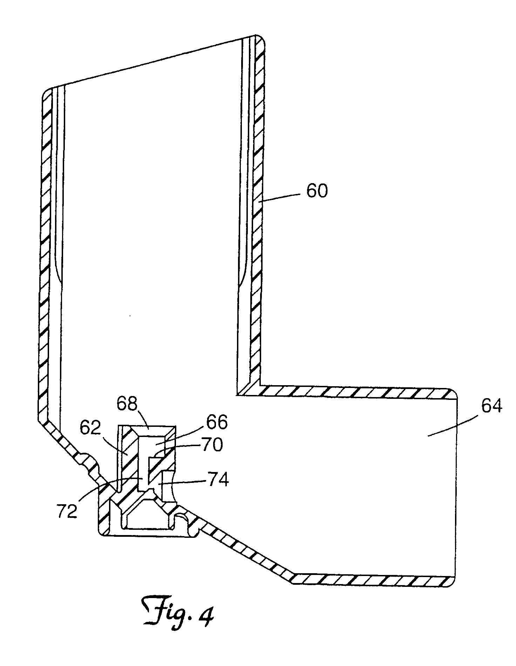

Figure 4 of the drawings represent a cross-section through a press-and-breathe actuator. The actuator comprises a casing (60) defining a body within which the aerosol container (not shown) is inserted, a nozzle block (62) and mouthpiece (64). The nozzle block (62) comprises an aperture (66) within which the valve stem is inserted. The opening of the aperture is chamfered at (68) to facilitate positioning of the valve stem. Within the aperture (66) is a ledge (70) upon which the end of the valve stem rests. The aperture (66) extends past the ledge (70) to form part of an expansion chamber (72) communicating with an orifice (74) which directs aerosol formulation towards the mouthpiece (64). When solution formulations are dispensed the orifice size is typically about 0.3mm in diameter.

It has been found that when deep drawn metal stems of the type shown in Figure 2 are used in metering valves of the type shown in Figure 1, there is a tendency for the metered dose inhalers to block to the extent that either no dose or a partial dose is delivered. The blockage tends to occur at. the side port of the valve stem and/or the orifice in the nozzle block of the actuator. The problem arises particularly when dispensing solution formulations comprising medicament dissolved in ethanol and hydrogen-containing fluorocarbon propellants, such as propellant 134a and/or propellant 227. A preferred example of such formulation comprises beclomethasone dipropionate as the medicament.

Investigations have revealed that after dispensing a dose of formulation residual ethanol is left inside the valve stem and nozzle block. The ethanol becomes saturated with the dissolved medicament and as the ethanol evaporates the medicament is precipitated and left behind as a solid deposit at the point where the ethanol has evaporated. While deposits of material on much of the interior of the valve stem do not immediately lead to blockage, the deposited medicament can dislodge, e.g. through vibration during handling or subsequent dose delivery or removal and replacement of the aerosol canister. There is a tendency for drug to build up in areas where it cannot be readily dislodged e.g. burrs and the curved portion at the outlet of the valve stem. Subsequent dislodgement of a large medicament build-up can cause complete blockage of the orifice of the actuator. Furthermore, the tendency for ethanol to evaporate is far higher at the openings to the atmosphere, namely, the valve stem side port and the actuator orifice. Thus, there is a natural tendency for medicament build-up in these regions and complete blockage of the side port has been observed.

Investigations have revealed that by reducing the "dead volume" of the valve stem the ethanol and medicament build-up can be substantially reduced. Thus, for example, sealing the interior of the valve stem in the region of the clotted line (46) would substantially eliminate dead-volume within the valve stem and thus the aerosol formulation would only contact and pass through those regions of the valve stem necessary to convey the formulation to the orifice of the actuator. However, it is not readily possible to seal a deep drawn valve stem in this region.

Attempts to remove the circumferential projection or burr from around the side port have not been successful. It is possible to insert a die prior to punching the side aperture which will minimise the formation of the burr. However, this technique substantially or completely eliminates the dishing effect on the outer surface around the side port which is obtained by punching without a die thereby leaving a sharp edge around the side port which is prone to damaging the diaphragm. Theoretically, this sharp edge may be removed by countersinking the outer rim of the aperture but it has proved not to be readily practical in view of the small thickness of the wall of the deep drawn valve stem. Furthermore, the wall of the valve stem is sufficiently thin that there is a tendency for the diaphragm to enter the side port and project beyond the inner wall of the valve stem which can result in damage to the diaphragm by the sharp inner edge of the side port.

Attempts to eliminate the curvature at the outlet end of the valve stem have also proved to be impractical. In order to facilitate assembly of the diaphragm on the valve stem it is necessary for the end of the valve stem to be chamfered or inwardly curved and the wall thickness of the deep drawn valve stem is not sufficient to form a reasonable chamfer. Also, since the end of the valve stem must also act as a bearing surface within the nozzle block of the actuator it is desirable not to reduce the area of the valve stem which contacts the nozzle block. Thus, it is not readily possible to completely eliminate the end curl of the deep drawn valve stem.

Figure 3 illustrates a valve stem suitable for use in the invention. The valve stem comprises an outer end (14), inner end (20), flange (26), groove (32), side port (34) and central channel (40). The side port and central channel are preferably formed by drilling and are arranged to minimise dead volume, i.e. the side port joins the outlet channel near the inner end of the outlet channel. Also, to avoid burrs, it is preferred to insert and remove the drill bit at least twice during the drilling operation.

The diameter of the central channel is selected such that the wall thickness of the valve stem is sufficient to provide the necessary strength characteristics and to provide a suitable bearing surface at the end of the valve stem whilst allowing for a chamfer or curvature (52) which facilitates assembly of the diaphragm. Although larger diameter outlet channels increase the surface area upon which ethanol and medicament may be deposited, a wide direct pathway from the side port to the nozzle block facilitates dispensing of the aerosol formulation.

The exterior of the side port (34) is countersunk at (54) so that a sharp edge is not presented to the diaphragm. The wall thickness of the valve stem is sufficiently large to prevent the diaphragm projecting beyond the internal surface of the valve stem. The internal surface of the valve stem is free from burrs around the side port. The cross-section of the outlet channel (40) is constant and circular and so does not provide any areas where there may be preferential build up of ethanol and medicament.

The valve stems according to the invention are preferably made of stainless steel. Stainless steel grade selection should be based not only on the machining characteristics, but also on the corrosion resistance of the material. Stainless steel grades with at least 11.5% chromium are preferred.

The exterior contours of the valve stem may be formed by machining and/or cold forging. Cold forging involves forcing a blank into a mould under pressure. The side port and outlet channel are then drilled.

The valve stems are preferably bright annealed after fabrication. Bright annealing is believed to improve the surface finish of the valve stem and thereby improve the frictional characteristics of the stem in relation to the diaphragm. Another benefit of bright annealing is that the improved surface finish of the interior surfaces may reduce the tendency for retention of ethanol and medicament. Bright annealing also has the advantage that it burns and removes any residual materials, such as machining fluids, to which the metal was exposed during fabrication.

Removal of such residuals, by bright annealing and/or special washing with, e.g., acids, is believed to actually improve corrosion resistance by enhancing the formation of a protective, passive chromium oxide film (referred to as "passivation"). Accordingly, the valve stems are preferably treated with an acid solution of nitric acid (at 5 to 50% concentration) or citric acid (1 to 10% concentration) or other appropriate compound to remove surface contamination so as to enhance spontaneous passivation.

In addition to the above, barreling or tumbling of the valve stems can also improve the corrosion resistance by enhancing the surface finish of the valve stem.

Typical dimensions for the outer end of the valve stem of the invention are:

| | |
|---|---|
| Outside diameter | 2.5 to 3mm, preferably about 2.8mm |
| diameter of outlet channel | 1.0 to 1.8mm, preferably about 1.2mm |
| length of outlet channel | 6 to 10mm, preferably about 8mm |
| diameter of side port | 0.4 to 0.6mm, preferably about 0.5mm |

The valves of the invention have been tested with aerosol formulations comprising a solution of medicament, beclomethasone dipropionate, hydrogen-containing fluorocarbon propellant (p134a, p227) and ethanol in varying amounts up to 20% by weight of the formulation and compared with valves having a deep drawn metal stem. The valves of the invention exhibited an extremely low and acceptable failure rate when dispensing 200 doses due to blockage of the side port or actuator orifice whereas the failure rate of the valves having deep drawn metal stems was many times in excess of the valves of the invention.

Particularly preferred formulations used with these valves of the invention are:

| | mg/ml | mg/ml |
|---|---|---|
| Beclomethasone Dipropionate | 1.00 | 2.00 |
| Ethanol | 94.80 | 94.72 |
| 1,1,1,2-tetrafluoroethane | 1090.20 | 1089.28 |
| Total | 1186.00 | 1186.00 |

## Claims

1. A metered dose aerosol product comprising:
an aerosol container having an aerosol formulation therein comprising medicament in one or more hydrogen-containing propellants;
said container being equipped with a valve comprising a metering chamber (12) and a valve stem (10) extending into the metering chamber (12) in sealing engagement therewith, the valve stem (10) having an outer end (14) projecting from the metering chamber (12), said outer end (14) having a central outlet channel (40) and a side port (34) in communication with the outlet channel (40), the valve stem (10) being movable between a non-dispensing position in which said side port (34) is located outside the metering chamber (12) and a dispensing position in which said side port (34) is located within the metering chamber (12) to facilitate dispensing of contents from the metering chamber (12) through said side port (34) and outlet channel (40); and
wherein at least said outer end (14) of the valve stem (10) is made of metal, said side port (34) joins the outlet channel (40) substantially at the inner end thereof and the outlet channel (40) has a constant cross-section throughout its length from the region of the side port (34) to its outlet (42), wherein the propellant is selected from propellant 134a (CF₃CH₂F), propellant 227 (CF₃CHFCF₃) and mixtures thereof, said aerosol formulation further comprises ethanol, and said medicament is dissolved in the formulation.

2. A metered dose aerosol product as claimed in Claim 1 in which the valve stem (10) comprises stainless steel.

3. A metered dose aerosol product as claimed in claim 2 in which the stainless steel contains at least 11.5% chromium.

4. A metered dose aerosol product as claimed in any preceding Claim in which the outlet channel (40) is formed by drilling.

5. A metered dose aerosol product as claimed in any preceding Claim in which the side port (34) is formed by drilling.

6. A metered dose aerosol product as claimed in Claim 5 in which the exterior of the side port (34) is countersunk.

7. A metered dose aerosol product as claimed in any preceding Claim in which the outer configuration of the valve stem (10) is obtained by machining.

8. A metered dose aerosol product as claimed in Claims 1 to 6 in which the outer configuration of the valve stem (10) is obtained by cold forging.

9. A metered dose aerosol product as claimed in any preceding Claim in which the valve stem (10) has been treated with a nitric acid solution.

10. A metered dose aerosol product as claimed in Claims 1-8 in which the valve stem (10) has been treated with a citric acid solution.

11. A metered dose aerosol product as claimed in any preceding Claim in which the valve stems (10) has been bright annealed.

12. A metered dose aerosol product as claimed in any preceding Claim in which the valve stem (10) has been barreled or tumbled to improve surface finish.

13. A metered dose aerosol product as claimed in any preceding Claim in which the dimensions of the outer end (14) of the valve stem (10) are:
| | |
|---|---|
| Outside diameter | 2.5 to 3mm |
| diameter of outlet channel (40) | 1.0 to 1.5mm |
| length of outlet channel (40) | 6 to 10mm |
| diameter of side port (34) | 0.4 to 0.6mm |

14. A metered dose aerosol product as claimed in Claim 12 in which the dimensions of the outer end (14) of the valve stem (10) are:
| | |
|---|---|
| Outside diameter | About 2.8mm |
| Diameter of outlet channel (40) | About 1.2mm |
| Length of outlet channel (40) | About 8mm |
| Diameter of side port (34) | About 0.5mm |

15. A metered dose aerosol product as claimed in any of Claims 1 to 14 in which the medicament is a steroid.

16. A metered dose aerosol product as claimed in Claim 15 in which the aerosol formulation comprises from 2 to 20% by weight ethanol.

17. A metered dose aerosol product as claimed in any preceding Claim in which the aerosol formulation comprises:
| | mg/ml |
|---|---|
| Beclomethasone Dipropionate | 1.00 |
| Ethanol | 94.80 |
| 1,1,1,2-tetrafluoroethane | 1090.20 |

18. A metered dose aerosol product as claimed in Claims 1-16 in which the aerosol formulation comprises:
| | mg/ml |
|---|---|
| Beclomethasone Dipropionate | 2.00 |
| Ethanol | 94.72 |
| 1,1,1,2-tetrafluoroethane | 1089.28 |

19. A metered dose aerosol product as claimed in any preceding Claim, further comprising an inhalation actuator comprising a nozzle block (62) and a mouthpiece (64), the nozzle block (62) defining an aperture (66) for accommodating the end of the valve stem (10) and an orifice (74) in communication with the aperture (66) directed towards the mouthpiece (64), the orifice (74) having a diameter of less than 0.4mm.

20. A metered dose aerosol product as claimed in Claim 19 in which the orifice (74) of the nozzle block (62) has a diameter of about 0.3mm.

## Patentansprüche

1. Ein Aerosoldosierprodukt mit:
einem Aerosolbehälter mit einer Aerosolformulierung mit einem Medikament in einem oder mehreren Treibmitteln, die Wasserstoff aufweisen;
wobei der Behälter mit einem Ventil mit Dosierkammer (12) und einem Ventilstößel (10) versehen ist, der sich in die Dosierkammer (12) erstreckt, wobei er damit im Dichtungsverbund ist, der Ventilstößel (10) mit einem Aussenende (14), das aus der Dosierkammer (12) hervorragt, das Aussenende (14) mit einem zentralen Auslasskanal (40) und einer Seitenöffnung (34), die mit dem Auslasskanal (40) in Verbindung steht, der Ventilstößel (10), der bewegbar ist zwischen einer Nichtabgabeposition, in der sich die Seitenöffnung (34) außerhalb der Dosierkammer (12) befindet, und einer Abgabeposition, in der sich, zur Erleichterung der Abgabe des Inhalts von der Dosierkammer (12) durch die Seitenöffnung (34) und den Auslasskanal (40), die Seitenöffnung (34) in der Dosierkammer (12) befindet; und
wobei mindestens das Aussenende (14) des Ventilstößels (10) aus Metall ist, die Seitenöffnung (34) den Auslasskanal (40) im wesentlichen mit dessen Innenende verbindet und der Auslasskanal (40) einen konstanten Querschnitt über seine gesamte Länge von dem Bereich der Seitenöffnung (34) zu dessen Auslass (42) hat, wobei das Treibgas von dem Treibgas 134a (CF₃CH₂F), Treibgas 227 (CF₃CHFCF₃) und Mischungen davon gewählt wird, die Aerosolformulierung des weiteren aus Ethanol besteht, und das Medikament in der Formulierung verteilt ist.

2. Aerosoldosierprodukt nach Anspruch 1, bei dem der Ventilstößel (10) Edelstahl aufweist.

3. Aerosoldosierprodukt nach Anspruch 2, bei dem der Edelstahl mindestens 11,5% Chrom hat.

4. Aerosoldosierprodukt nach einem der vorhergehenden Ansprüche, bei dem der Auslasskanal (40) durch Bohren ausgebildet ist.

5. Aerosoldosierprodukt nach einem der vorhergehenden Ansprüche, bei dem die Seitenöffnung (34) durch Bohren ausgebildet ist.

6. Aerosoldosierprodukt nach Anspruch 5, bei dem das Äußere der Seitenöffnung (34) angesenkt ist.

7. Aerosoldosierprodukt nach einem der vorhergehenden Ansprüche, bei dem die Außenkonfiguration des Ventilstößels (10) durch maschinelle Bearbeitung erreicht wird.

8. Aerosoldosierprodukt nach Anspruch 1 bis 6, bei dem die Außenkonfiguration des Ventilstößels (10) durch Kaltverformung erreicht wird.

9. Aerosoldosierprodukt nach einem der vorhergehenden Ansprüche, bei dem der Ventilstößel (10) mit einer Stickstoffsäurelösung behandelt worden ist.

10. Aerosoldosierprodukt nach Ansprüchen 1 bis 8, bei dem der Ventilstößel (10) mit einer Zitronensäurelösung behandelt worden ist.

11. Aerosoldosierprodukt nach einem der vorhergehenden Ansprüche, bei dem der Ventilstößel (10) blankgeglüht worden ist.

12. Aerosoldosierprodukt nach einem der vorhergehenden Ansprüche, bei dem der Ventilstößel (10) zur Verbesserung der Oberflächenbeschaffenheit poliert oder durch Trommelbehandlung bearbeitet worden ist.

13. Aerosoldosierprodukt nach einem der vorhergehenden Ansprüche, in dem die Ausmaße des Aussenendes (14) des Ventilstößels (10) sind:
| | |
|---|---|
| Äußerer Durchmesser | 2,5 bis 3 mm |
| Durchmesser des Auslasskanals (40) | 1,0 bis 1,5 mm |
| Länge des Auslasskanals (40) | 6 bis 10 mm |
| Durchmesser der Seitenöffnung (34) | 0,4 bis 0,6 mm |

14. Aerosoldosierprodukt nach Anspruch 12, bei dem die Ausmaße des Außenendes (14) des Ventilstößels (10) sind:
| | |
|---|---|
| Äußerer Durchmesser | Ungefähr 2,8 mm |
| Durchmesser des Auslasskanals (40) | Ungefähr 1,2 mm |
| Länge des Auslasskanals (40) | Ungefähr 8 mm |
| Durchmesser der Seitenöffnung (34) | Ungefähr 0,5 mm |

15. Aerosoldosierprodukt nach einem der Ansprüche 1 bis 14, bei dem das Medikament ein Steroid ist.

16. Aerosoldosierprodukt nach Anspruch 15, bei dem die Aerosolformulierung 2 bis 20 Gewichtsprozent Ethanol aufweist.

17. Aerosoldosierprodukt nach einem vorhergehenden Anspruch, bei dem die Aerosolformulierung aufweist:
| | mg/ml |
|---|---|
| Beclomethasone Dipropionat | 1,00 |
| Ethanol | 94,80 |
| 1, 1, 1, 2-Tetrafluorethan | 1090,20 |

18. Aerosoldosierprodukt nach Ansprüchen 1 bis 16, bei dem die Aerosolformulierung aufweist:
| | mg/ml |
|---|---|
| Beclomethasone Dipropionat | 2,00 |
| Ethanol | 94,72 |
| 1, 1, 1, 2-Tetrafluorethan | 1089,28 |

19. Aerosoldosierprodukt nach einem der vorhergehenden Ansprüche, des weiteren aufweisend ein Inhalationsbetätigungsglied mit einem Düseneinsatz (62) und einem Mundstück (64), wobei der Düseneinsatz (62) eine Öffnung (66) zur Unterbringung des Endes des Ventilstößels (10) und eine Mündung (74) definiert, die mit der Öffnung (66) in Verbindung steht, die zum Mundstück (64) ausgerichtet ist und die Mündung (74) einen Durchmesser von weniger als 0,4 mm hat.

20. Aerosoldosierprodukt nach Anspruch 19, in dem die Austrittsöffnung (74) des Düseneinsatzes (62) einen Durchmesser von ungefähr 0,3 mm hat.

## Revendications

1. Produit aérosol dosé, comportant :
un conteneur d'aérosol ayant une formulation d'aérosol dans celui-ci comportant un médicament contenu dans un ou plusieurs agents propulseurs contenant de l'hydrogène,
ledit conteneur étant muni d'une soupape comportant une chambre de dosage (12) et une tige de soupape (10) s'étendant dans la chambre de dosage (12), mise en contact étanche avec celle-ci, la tige de soupape (10) ayant une extrémité extérieure (14) faisant saillie à partir de la chambre de dosage (12), ladite extrémité extérieure (14) ayant un canal de sortie central (40) et un orifice latéral (34) en communication avec le canal de sortie (40), la tige de soupape (10) étant mobile entre une position de non distribution dans laquelle ledit orifice latéral (34) est positionné à l'extérieur de la chambre de dosage (12) et une position de distribution dans laquelle ledit orifice latéral (34) est positionné dans la chambre de dosage (12) pour faciliter la distribution du contenu à partir de la chambre de dosage (12) à travers ledit orifice latéral (34) et ledit canal de sortie (40), et
dans lequel au moins ladite extrémité de sortie (14) de la tige de soupape (10) est constituée d'un métal, ledit orifice latéral (34) rejoint le canal de sortie (40) sensiblement au niveau de son extrémité intérieure et le canal de sortie (40) a une coupe transversale constante sur toute sa longueur allant de la zone de l'orifice latéral (34) jusqu'à sa sortie (42), dans lequel l'agent propulseur est choisi parmi un agent propulseur 134a (CF₃CH₂F), un agent propulseur 227 (CF₃CHFCF₃) et des mélanges de ceux-ci,
ladite formulation d'aérosol comporte de plus de l'éthanol, et
ledit médicament est dissous dans la formulation.

2. Produit aérosol dosé selon la revendication 1, dans lequel la tige de soupape (10) est constituée d'acier inoxydable.

3. Produit aérosol dosé selon la revendication 2, dans lequel l'acier inoxydable contient au moins 11,5 % de chrome.

4. Produit aérosol dosé selon l'une quelconque des revendications précédentes, dans lequel le canal de sortie (40) est formé par perçage.

5. Produit aérosol dosé selon l'une quelconque des revendications précédentes, dans lequel l'orifice latéral (34) est formé par perçage.

6. Produit aérosol dosé selon la revendication 5, dans lequel l'extérieur de l'orifice latéral (34) est fraisé.

7. Produit aérosol dosé selon l'une quelconque des revendications précédentes, dans lequel la configuration extérieure de la tige de soupape (10) est obtenue par usinage.

8. Produit aérosol dosé selon l'une quelconque des revendications 1 à 6, dans lequel la configuration extérieure de la tige de soupape (10) est obtenue par forgeage à froid.

9. Produit aérosol dosé selon l'une quelconque des revendications précédentes, dans lequel la tige de soupape (10) a été traitée avec une solution d'acide nitrique.

10. Produit aérosol dosé selon l'une quelconque des revendications 1 à 8, dans lequel la tige de soupape (10) a été traitée avec une solution d'acide citrique.

11. Produit aérosol dosé selon l'une quelconque des revendications précédentes, dans lequel la tige de soupape (10) a subi un recuit blanc.

12. Produit aérosol dosé selon l'une quelconque des revendications précédentes, dans lequel la tige de soupape (10) a été traitée au tambour ou au cylindre pour améliorer le fini de surface.

13. Produit aérosol dosé selon l'une quelconque des revendications précédentes, dans lequel les dimensions de l'extrémité extérieure (14) de la tige de soupape (10) sont :
| | |
|---|---|
| Diamètre extérieur | 2,5 à 3 mm |
| Diamètre de canal de sortie (40) | 1,0 à 1,5 mm |
| Longueur de canal de sortie (40) | 6 à 10 mm |
| Diamètre d'orifice latéral (34) | 0,4 à 0,6 mm |

14. Produit aérosol dosé selon la revendication 12, dans lequel les dimensions de l'extrémité extérieure (14) de la tige de soupape (10) sont :
| | |
|---|---|
| Diamètre extérieur | Environ 2,8 mm |
| Diamètre de canal de sortie (40) | Environ 1,2 mm |
| Longueur de canal de sortie (40) | Environ 8 mm |
| Diamètre d'orifice latéral (34) | Environ 0,5 mm |

15. Produit aérosol dosé selon l'une quelconque des revendications 1 à 14, dans lequel le médicament est un stéroïde.

16. Produit aérosol dosé selon la revendication 15, dans lequel la formulation aérosol comporte de 2 à 20 % en poids d'éthanol.

17. Produit aérosol dosé selon l'une quelconque des revendications précédentes, dans lequel la formulation d'aérosol comporte :
| | mg/ml |
|---|---|
| Béclométhasone dipropionate | 1,0 |
| Ethanol | 94,80 |
| 1,1,1,2-tétrafluroéthane | 1090,20 |

18. Produit aérosol dosé selon l'une quelconque des revendications 1 à 16, dans lequel la formulation d'aérosol comporte :
| | mg/ml |
|---|---|
| Béclométhasone dipropionate | 2,0 |
| Ethanol | 94,72 |
| 1,1,1,2-tétrafluroéthane | 1089,28 |

19. Produit aérosol dosé selon l'une quelconque des revendications précédentes, comportant un actionneur d'inhalation comportant un bloc de buse (62) et une pièce de bouche (64), le bloc de buse (62) définissant une ouverture (66) pour recevoir l'extrémité de la tige de soupape (10) et un orifice (74) en communication avec l'ouverture (66) dirigé en direction de la pièce de bouche (64), l'orifice (74) ayant un diamètre inférieur à 0,4 mm.

20. Produit aérosol dosé selon la revendication 19, dans lequel l'orifice (74) du bloc de buse (62) a un diamètre d'environ 0,3 mm.
